# FASCICULE DE BREVET EUROPEEN

(11) **EP 2 117 597 B1**
(45) Date de publication et mention de la délivrance du brevet: **01.11.2017**
(21) Numéro de dépôt: 08775559.1
(22) Date de dépôt: 15.02.2008
(51) Int. Cl.: A61K 47/06, A61K 47/36, A61K 47/44

(54) **COMPOSITION PHARMACEUTIQUE DE TYPE EMULSION HUILE DANS HUILE**
PHARMAZEUTISCHE ZUSAMMENSETZUNG IN FORM EINER ÖL-IN-ÖL-EMULSION
OIL-IN-OIL EMULSION-TYPE PHARMACEUTICAL COMPOSITION

(30) Priorité: 15.02.2007 FR 0701114
(43) Date de publication de la demande: 18.11.2009
(73) Titulaire: Saintrond, Alain, 28100 Dreux (FR)
(72) Inventeur: ALLART, Jean-Claude, F-62520 Le Touquet (FR); LEFEVRE, Jean-Marie, F-80000 Amiens (FR); PEYROT, Jacques, F-63000 Clermont-ferrand (FR); MARTY, Jean-Paul, F-83650 Les Adrets De L'esterel (FR); SAINTROND, Alain, F-28100 Dreux (FR)
(74) Mandataire: L'Helgoualch, Jean
(86) Numéro de dépôt international: PCT/FR2008/000201
(87) Numéro de publication internationale: WO 2008/129153

(56) Documents cités:
- WO-A-03/000396
- DE-A1- 19 826 503
- DE-A1- 19 907 305
- FR-A1- 2 694 194
- EGAWA Y ET AL: "Oil-based cosmetics such as lipstick, lip cream and foundation, comprises preset amount of perfluoro-alkyl-polyalkyl siloxy silicic acid, silicone gum, coloring material and iso dodecane" WPI / THOMSON, 13 février 2003 (2003-02-13), XP002437902
- PASARIN ZARAUZA L: "Hand and fingernail cream, comprises sunflower oil, castor oil, glycerin, vaseline, aloe vera gel, lemon extract and cornflour" DERWENT, 16 mars 2001 (2001-03-16), XP002330463
- COSMO CHEM SARL: "MicroMatrix"[Online] 2004, XP002455402 Extrait de l'Internet: URL:www.micromatrix.info/fr/novelty.html> [extrait le 2007-10-17]
- COSMO CHEM SARL: "Creagel crystal"[Online] 2004, XP002455506 Extrait de l'Internet: URL:www.creationscouleurs.com/fr/products/ cgelcr.html> [extrait le 2007-10-18]

## Description

L'invention concerne une composition pharmaceutique de type émulsion huile dans huile, ainsi que son utilisation.

Cette composition est plus particulièrement destinée à être utilisée dans le cadre d'un traitement contre la sécheresse vaginale chez la femme, qui peut être liée à différents facteurs, isolés ou associés. Elle peut résulter par exemple de modifications du statut hormonal dues à la ménopause ou à un traitement hormonal à visée contraceptive ou thérapeutique.

Pour traiter ce problème, on connaît déjà plusieurs produits lubrifiants qui comprennent, en tant que partie active, des composants tels que le gel de paraffine (ou Vaseline®), la cellulose et ses dérivés, et/ou des carboxypolyméthylènes. Toutefois, chacun de ces composants présente des inconvénients. Le gel de paraffine utilisé seul a un effet occlusif susceptible d'entraîner un oedème intercellulaire et cellulaire et de déclencher des réactions cytokiniques inflammatoires. La cellulose et ses dérivés peuvent donner lieu à des irritations car la structure en réseau des fibres de cellulose crée des spicules qui agressent mécaniquement la partie superficielle de la muqueuse. Les carboxypolyméthylènes modifient la pression osmotique et l'activité des pompes à calcium, ce qui influence le pH et la résistivité électrique de la muqueuse et peut aggraver les hyperesthésies.

En outre, les produits actuellement sur le marché comprennent des conservateurs parmi lesquels on trouve des parabens à longue chaîne et des isothiazolinones, qui sont soupçonnés d'avoir des effets allergisants et d'être impliqués dans des résorptions systémiques agissant sur les récepteurs hormonaux oestrogéniques.

Tous ces produits lubrifiants présentent également l'inconvénient de devoir être appliqués très fréquemment, car leur effet disparaît au bout de quelques heures (6 à 8 heures au maximum).

Il existe par conséquent un besoin important de mettre au point un nouveau produit ne présentant pas les inconvénients précités, et dont l'efficacité est accrue par rapport aux produits déjà existants.

On connaît dans la technique des formulations, généralement sous forme d'émulsions, où des principes actifs sont incorporés et isolés les uns des autres pour éviter des interactions, ou pour contrôler leur libération dans le temps. Ainsi la demande WO 03000396 décrit des émulsions huile dans huile à base de silicone, c'est-à-dire comprenant deux liquides non aqueux et non miscibles dont l'un est une silicone. Ces émulsions peuvent être à base de diméthicone et d'huile d'origine animale ou végétale, de préférence stabilisées par addition d'un copolymère, et elles sont utiles pour protéger des matières actives hydrophobes sensibles à l'oxydation telles que des vitamines, des filtres UV, des antimicrobiens ou des parfums. Le brevet FR 2694194 décrit un gel aqueux hydratant destiné au traitement de la sécheresse vaginale, ayant une teneur en eau d'au moins 80% et comprenant un polysaccharide et un agent hydratant.

La présente invention a pour objet une composition pharmaceutique dont la combinaison de composants permet de procurer à l'utilisatrice non seulement un effet lubrifiant, mais également un effet hydratant et anti-inflammatoire, et ce pendant une durée plus longue que les produits connus.

A cet effet, l'invention a pour objet une composition pharmaceutique de type émulsion huile dans huile telle que définie dans la revendication 1 qui comprend :
- un mélange de glycérine et de gel de paraffine ;
- une ou plusieurs huiles perfluorées ;
- des lipides gélifiés ;
- des polysaccharides encapsulant la ou les huiles perfluorées et les lipides gélifiés.

Cette composition constitue une émulsion multiple huile dans huile purement lipidique, c'est-à-dire sans eau, qui contient des lipides particuliers ne se solubilisant pas les uns dans les autres. Chaque catégorie de lipides présente des propriétés utiles pour l'effet recherché, l'action de chaque catégorie de lipides étant en outre optimisée par sa formulation au sein du mélange.

Le mélange de glycérine et de gel de paraffine (ou Vaseline®), procure un effet lubrifiant immédiat. Le fait d'associer le gel de paraffine à de la glycérine, permet en outre de réduire l'effet occlusif du gel de paraffine évoqué plus haut. Selon une réalisation préférentielle, le mélange de gel de paraffine et de glycérine est présent dans une proportion comprise entre 10 et 80% en poids par rapport au poids total de la composition, et en particulier entre 40 et 50% en poids par rapport au poids total de la composition.

Les polysaccharides qui encapsulent la ou les huiles perfluorées et les lipides gélifiés forment sur la muqueuse un film qui s'adapte parfaitement à la topographie de celle-ci. Ce film qui retient les composés actifs permet d'obtenir une libération prolongée et contrôlée des lipides encapsulés, en fixant le mélange de lipides sur la surface de la muqueuse, ce qui rend la lubrification particulièrement efficace. L'effet de la composition selon l'invention subsiste ainsi localement et efficacement pendant 2 à 4 jours.

La présence d'un film de polysaccharides sur la muqueuse vaginale a en outre pour avantage de procurer une hydratation à long terme et de favoriser la cicatrisation. Cette hydratation prolongée de la muqueuse lui permet de récupérer sa capacité d'auto-lubrification dont l'effet sera synergique avec l'effet lubrifiant de la composition.

Les polysaccharides utilisés dans l'invention sont choisis parmi ceux possédant de bonnes propriétés filmogènes et lubrifiantes, présentant une parfaite innocuité pour la muqueuse vaginale, et capables d'encapsuler des huiles perfluorées et des lipides. On utilise des polysaccharides sous forme de microparticules à structure de matrice polymère où des liaisons covalentes sont formées entre les chaînes de polysaccharides, permettant l'encapsulation des huiles perfluorées et des lipides. Les polysaccharides (ou polyosides) utilisés dans l'invention peuvent être ioniques ou non ioniques, la seule condition étant qu'ils possèdent une structure permettant d'encapsuler les huiles perfluorées et les lipides et qu'ils soient biocompatibles. On peut utiliser par exemple des polysaccharides tels que des dérivés de β-cyclodextrine, l'acide hyaluronique, le chitosane ou un alginate. Plus particulièrement, on peut utiliser le composé anionique d'origine végétale à haut poids moléculaire commercialisés sous le nom Glycofilm® (Solabia) et le composé MicroMatrix® (société Créations Couleurs).

Les huiles perfluorées, outre un effet lubrifiant, présentent des propriétés de transport de gaz, en particulier l'oxygène, ce qui permet d'assurer une bonne oxygénation de la muqueuse et d'améliorer ainsi sa souplesse et son hydratation. Ces composés perfluorés présentent également des effets diélectriques qui confèrent à la composition selon l'invention des propriétés anti-inflammatoires.

La ou les huiles perfluorées sont avantageusement présentes dans une proportion comprise entre 1 et 15% en poids par rapport au poids total de la composition, et de préférence entre 10 et 15% en poids. On peut prévoir que les huiles perfluorées comprennent entre 15% et 50% de composés à chaîne longue. La longueur des chaînes détermine la demi-vie des produits : plus les chaînes sont longues, plus l'effet conféré par les huiles persistera. Comme exemple d'huile perfluorée utilisable dans l'invention, on peut citer les perfluorodécalines, et notamment les produits Fiflow® commercialisées par la société Créations Couleurs.

Les lipides gélifiés utilisés sont choisis non seulement pour leurs propriétés lubrifiantes, mais également pour leur effet émollient. Ces lipides gélifiés sont par exemple des huiles synthétiques ou naturelles, telles que le polydécène hydrogéné, les polyisobutènes, le Meadowfoam Seed Oil, l'huile d'olive, l'huile de tournesol, l'huile d'amande douce, des esters d'acides gras tels que le palmitate d'isopropyle, le myristate d'isopropyle, l'isostearate d'isopropyle, l'isostearate d'isostéaryle, le sébacate de diisopropyle, le dipelargonate de propylène, le dipelargonate de propylène, le 2-éthylhexyl isononoate, le 2-éthyl-hexylstéarate, les lactates d'alcools gras comprenant de 12 à 16 atomes de carbone tels que le lactate cétylique, le lactate de lauryle, le lanolate d'isopropyle, le 2-éthylhexyl salicylate, le myristate cétylique, le myristate oléylique, le stéarate oléylique, l'oléate oléylique, le laurate d'hexyle et le laurate d'isohexyle, l'isohexadécane, ces huiles étant épaissies par polymérisation. En particulier, on peut citer les lipides Creagel®, commercialisés par la société Créations Couleurs. Ces lipides gélifiés sont présents de préférence dans une proportion comprise entre 1% et 30% en poids par rapport au poids total de la composition, et plus particulièrement entre 5 et 15% par rapport au poids total de la composition.

De façon avantageuse, la composition selon l'invention comprend en outre une ou plusieurs silicones, par exemple la diméthicone et la cyclométhicone, isolément ou en mélange, encapsulées dans les polysaccharides. Cette addition de silicones contribue à augmenter l'effet lubrifiant de la composition tout en lui conférant un effet adoucissant.

Selon une réalisation préférentielle, les silicones sont présentes dans une proportion comprise entre 1% et 5% en poids par rapport au poids total de la composition.

La composition peut également contenir une substance sous forme de poudre très fine, la lauroyl lysine, qui est encapsulée dans les polysaccharides et a pour effet de diminuer le coefficient de friction de la formulation. Cette substance est commercialisée sous le nom Amihope® par la société AminoSciences.

On peut prévoir que la lauroyl lysine soit présente dans une proportion comprise entre 0,1% et 5% en poids par rapport au poids total de la composition.

La composition comprend également de préférence un système conservateur faisant partie des produits listés et autorisés.

La composition à base d'émulsion huile dans huile de la présente invention peut être préparée de manière classique, par exemple en mélangeant les polysaccharides, les lipides gélifiés et les huiles perfluorées, puis la glycérine et le gel de paraffine, et en ajoutant les additifs précités, sous agitation à température ambiante jusqu'à obtenir une émulsion homogène.

La composition selon l'invention procure donc une lubrification immédiate, obtenue par le mélange de glycérine et de gel de paraffine, qui est prolongée par la libération progressive d'autres lipides fixés sur le film formé par les polysaccharides, ces lipides combinant des effets lubrifiants, hydratants et anti-inflammatoires.

La composition selon l'invention est particulièrement destinée à la fabrication d'un médicament ou d'un produit à usage topique pour le traitement de la sécheresse vaginale. Elle permet l'obtention d'un produit d'emploi aisé, facilement toléré, et dont les applications peuvent être espacées tout en procurant à l'utilisatrice un confort permanent. Ainsi d'excellents résultats sont obtenus avec une seule application par jour dans les cas les plus difficiles, jusqu'à une application par semaine.

Les indications d'un tel produit comprenant la composition selon l'invention sont le traitement de la sécheresse vaginale chez la femme avant ou après la ménopause, qui peut résulter d'une modification hormonale, de soins d'hygiène inadaptés (douches trop chaudes, utilisation de produits antiseptiques moussants mal rincés), d'infections vulvo-vaginales, d'infections urinaires répétées, de traitements anti-levuriques fréquents et prolongés, ou encore de dermatoses à localisation vulvaire (psoriasis, eczéma sec, lichen scléro-atropique, lichénifications, névrodermites, prurit vulvaire, vestibulites).

On décrit à présent plusieurs exemples de compositions selon l'invention, dans lequel les pourcentages indiqués sont des pourcentages en poids par rapport au poids total de la composition.

### Exemple 1

| | |
|---|---|
| Vaseline® | 25% |
| Glycérine | 25% |
| Polyisobutène hydrogéné | 2% |
| Diméthicone | 5% |
| Isohexadécane | 1% |
| Ethylhexyl isononoate | 1% |
| Copolymère éthylène-propylène | 14,5% |
| Polyéthylacrylate | 1% |
| Perfluoroperhydrophenantrène | 3% |
| Perfluorodécaline | 2% |
| Perfluorodiméthylcyclohexane | 6% |
| Perfluorohexane | 2% |
| Perfluoropolyméthylisopropyléther | 2% |
| Lauroyl lysine | 5% |
| Polysaccharides (MicroMatrix®) | 5% |
| Système conservateur | 0,5% |

### Exemple 2

| | |
|---|---|
| Vaseline® | 25% |
| Glycérine | 25% |
| Polydécène hydrogéné | 5% |
| Diméthicone | 5% |
| Isohexadécane | 1% |
| Ethylhexyl isononoate | 1% |
| Copolymère éthylène-propylène | 14% |
| Polyéthylacrylate | 1% |
| Perfluoroperhydrophenantrène | 2% |
| Perfluorodécaline | 2% |
| Perfluorodiméthylcyclohexane | 3% |
| Perfluorohexane | 3% |
| Perfluoropolyméthylisopropyléther | 2,5% |
| Lauroyl lysine | 5% |
| Polysaccharides | 5% |
| Système conservateur | 0,5% |

Suivant les techniques classiques connues de l'homme du métier, ces compositions sont préparées en mélangeant les phases de manière à bien homogénéiser l'ensemble, de sorte à obtenir une émulsion multiple.

## Revendications

1. Composition pharmaceutique de type émulsion huile dans huile, **caractérisée en ce qu'**elle comprend :
- un mélange de glycérine et de gel de paraffine ;
- une ou plusieurs huiles perfluorées ;
- des lipides gélifiés choisis parmi les huiles synthétiques ou naturelles, les huiles étant épaissies par polymérisation ;
- des polysaccharides sous forme de microparticules à structure de matrice polymère à liaisons covalentes formées entre les chaînes de polysaccharides, encapsulant la ou les huiles perfluorées et les lipides gélifiés.

2. Composition selon la revendication 1, **caractérisée en ce qu'**elle comprend en outre une ou plusieurs silicones, isolément ou en mélange, encapsulées dans lesdits polysaccharides.

3. Composition selon la revendication 2, **caractérisée en ce que** la silicone est choisie parmi la cyclométhicone et la diméthicone isolément ou en mélange.

4. Composition selon l'une quelconque des revendications 1 à 3, **caractérisée en ce qu'**elle comprend en outre de la lauroyl lysine encapsulée dans lesdits polysaccharides.

5. Composition selon l'une quelconque des revendications 1 à 4, **caractérisée en ce que** le mélange de gel de paraffine et de glycérine est présent dans une proportion comprise entre 10 et 80% en poids par rapport au poids total de la composition.

6. Composition selon l'une quelconque des revendications 1 à 5, **caractérisée en ce que** la ou les huiles perfluorées sont présentes dans une proportion comprise entre 1 et 15% en poids par rapport au poids total de la composition.

7. Composition selon l'une quelconque des revendications 1 à 6, **caractérisée en ce que** la ou les huiles perfluorées comprennent des perfluorodécalines.

8. Composition selon l'une quelconque des revendications 1 à 7, **caractérisée en ce que** lesdits lipides gélifiés sont présents dans une proportion comprise entre 1% et 30% en poids par rapport au poids total de la composition.

9. Composition selon la revendication 2 ou 3, **caractérisée en ce que** les silicones sont présentes dans une proportion comprise entre 1% et 5% en poids par rapport au poids total de la composition.

10. Composition selon la revendication 4, **caractérisée en ce que** la lauroyl lysine est présente dans une proportion comprise entre 0,1% et 5% en poids par rapport au poids total de la composition.

11. Composition selon l'une quelconque des revendications 1 à 10 pour utilisation à titre de médicament à usage topique destiné au traitement de la sécheresse vaginale.

## Patentansprüche

1. Pharmazeutische Zusammensetzung vom Typ Öl-in-Öl-Emulsion, **dadurch gekennzeichnet, dass** sie umfasst:
- ein Gemisch aus Glycerin und Paraffingel,
- ein oder mehrere perfluorierte Öle,
- gelierte Lipide, ausgewählt aus den synthetischen oder natürlichen Ölen, wobei die Öle durch Polymerisation verdickt sind,
- Polysaccharide in Form von Mikropartikeln mit Polymermatrixstruktur mit kovalenten Bindungen zwischen den Polysaccaridketten, welche das oder die perfluorierten Öle und die gelierten Lipide einkapseln.

2. Zusammensetzung nach Anspruch 1, **dadurch gekennzeichnet, dass** sie ferner ein oder mehrere Silikone, isoliert oder im Gemisch, eingekapselt in die Polysaccharide, umfasst.

3. Zusammensetzung nach Anspruch 2, **dadurch gekennzeichnet, dass** das Silikon aus dem Cyclomethicon und dem Dimethicon, isoliert oder im Gemisch, ausgewählt ist.

4. Zusammensetzung nach einem der Ansprüche 1 bis 3, **dadurch gekennzeichnet, dass** sie ferner Lauroyllysin, eingekapselt in die Polysaccharide, umfasst.

5. Zusammensetzung nach einem der Ansprüche 1 bis 4, **dadurch gekennzeichnet, dass** das Gemisch aus Paraffingel und Glycerin und/oder ihren Derivate in einem Anteil zwischen 10 und 80 Gew.-% inklusive in Bezug zum Gesamtgewicht der Zusammensetzung vorhanden ist.

6. Zusammensetzung nach einem der Ansprüche 1 bis 5, **dadurch gekennzeichnet, dass** das oder die perfluorierten Öle in einem Anteil zwischen 1 und 15 Gew.-% inklusive in Bezug zum Gesamtgewicht der Zusammensetzung vorhanden sind.

7. Zusammensetzung nach einem der Ansprüche 1 bis 6, **dadurch gekennzeichnet, dass** das oder die perfluorierten Öle Perfluordecalin enthalten.

8. Zusammensetzung nach einem der Ansprüche 1 bis 7, **dadurch gekennzeichnet, dass** die gelierten Lipide in einem Anteil zwischen 1 und 30 Gew.-% inklusive in Bezug zum Gesamtgewicht der Zusammensetzung vorhanden sind.

9. Zusammensetzung nach Anspruch 2 oder 3, **dadurch gekennzeichnet, dass** die Silikone in einem Anteil zwischen 1 und 5 Gew.-% inklusive in Bezug zum Gesamtgewicht der Zusammensetzung vorhanden sind.

10. Zusammensetzung nach Anspruch 4, **dadurch gekennzeichnet, dass** das Lauroyllysin in einem Anteil zwischen 0,1 und 5 Gew.-% inklusive in Bezug zum Gesamtgewicht der Zusammensetzung vorhanden ist.

11. Verwendung einer Zusammensetzung nach einem der Ansprüche 1 bis 10 zwecks Verwendung als Arzneimittel zur topischen Anwendung zur Behandlung von Scheidentrockenheit.

## Claims

1. Pharmaceutical composition of oil-in-oil emulsion type, **characterized in that** it comprises:
- a mixture of glycerine and paraffin gel;
- one or more perfluorinated oils;
- gelled lipids selected from among synthetic or natural oils, the oils being thickened by polymerization;
- polysaccharides in the form of microparticles having a polymer matrix structure with covalent bonds formed between the polysaccharide chains, encapsulating the perfluorinated oil(s) and gelled lipids.

2. The composition according to claim 1, **characterized in that** it further comprises one or more silicones, alone or in a mixture, encapsulated in said polysaccharides.

3. The composition according to claim 2, **characterized in that** the silicone is selected from among cyclomethicone and dimethicone, alone or in a mixture.

4. The composition according to any of claims 1 to 3, **characterized in that** it further comprises lauroyl lysine encapsulated in said polysaccharides.

5. The composition according to any of claims 1 to 4, **characterized in that** the mixture of paraffin gel and glycerine is contained in a proportion of between 10 and 80 % by weight relative to the total weight of the composition.

6. The composition according to any of claims 1 to 5, **characterized in that** the perfluorinated oil(s) are contained in a proportion of between 1 and 15 % by weight relative to the total weight of the composition.

7. The composition according to any of claims 1 to 6, **characterized in that** the perfluorinated oil(s) comprise perfluorodecalins.

8. The composition according to any of claims 1 to 7, **characterized in that** said gelled lipids are contained in a proportion of between 1 % and 30 % by weight relative to the total weight of the composition.

9. The composition according to claim 2 or 3, **characterized in that** the silicones are contained in a proportion of between 1 % and 5 % by weight relative to the total weight of the composition.

10. The composition according to claim 4, **characterized in that** the lauroyl lysine is contained in a proportion of between 0.1 % and 5 % by weight relative to the total weight of the composition.

11. The composition according to any of claims 1 to 10 for use as medicinal product for topical use intended for the treatment of vaginal dryness.
